Europäisches Patentamt

European Patent Office

Office européen des brevets

(11) Publication number: **0 047 042**
**B1**

## (12) EUROPEAN PATENT SPECIFICATION

(45) Date of publication of patent specification: **31.07.85**

(21) Application number: **81200955.3**

(22) Date of filing: **28.08.81**

(51) Int. Cl.⁴: **A 61 M 5/315**

(54) **Injection syringe.**

(30) Priority: **29.08.80 NL 8004903**

(43) Date of publication of application:
**10.03.82 Bulletin 82/10**

(45) Publication of the grant of the patent:
**31.07.85 Bulletin 85/31**

(84) Designated Contracting States:
**AT BE CH DE FR GB IT LI LU NL SE**

(56) References cited:
**DE-U-8 009 848**
**FR-A-1 242 553**
**FR-A-1 348 320**
**FR-A-2 224 174**
**GB-A- 447 741**
**US-A-3 672 369**

(73) Proprietor: **Coöperatieve Apothekers**
**Vereeniging "De Onderlinge Pharmaceutische**
**Groothandel" U.A.**
**Europalaan 2**
**NL-3500 GB Utrecht (NL)**

(72) Inventor: **Muller, Robert Hans**
**Ijsselsteinseweg 42**
**NL-3435 VH Nieuwegein (NL)**

(74) Representative: **Kooy, Leendert Willem et al**
**OCTROOIBUREAU VRIESENDORP & GAADE**
**P.O. Box 266**
**NL-2501 AW The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Description

The present invention relates to a hypodermic syringe provided with a container with a hollow adapter connected to the container on which a hypodermic needle can be placed, and with a piston slidable in the container which piston is provided at its upper side with a closed projecting part tapering in the direction of the hypodermic needle to be placed on the adapter, which projecting part in the extreme position of the piston in the container fills up the cavity in the adapter to leave unoccupied only a small space between the inner wall of the adapter and the outer wall of said projection, the top of the projection then closing the passage of the hollow adapter to the hypodermic needle.

It is generally known that such hypodermic syringes can be applied for medical purposes. In many cases they are intended for being used once only, so for injecting a dose of a medical liquid, for instance an insulin liquid generally.

The liquid which remains in the hollow adapter gets lost. This is a drawback when expensive medical liquids or those in short supply are concerned. This is in particular the case with insulin liquids, because insulin is recovered from animal organs, of which only a limited supply is available. On the other hand the number of diabetes patients has been steadily increased of recent years.

Despite it, in the application of each dose of 1 ml at an average of for instance an insulin liquid about 9% of the liquid gets lost in the hollow adapter. For the volume of the hollow space in the hollow adapter cannot be made as small as possible, because air must be able to escape past it before the liquid is injected into the patient. This air which is already present in the unused needle and syringe and which adheres to the walls and the little piston of the hypodermic syringe is removed for the major part as follows; the filled syringe is kept with the needle upwardly and one taps to the hypodermic syringe. By so doing the air rises and escapes from the hypodermic syringe, provided the passage in the hollow adapter is not too small.

DE—U—8 009 848 describes a syringe in which the loss of injection liquid is diminished by using a piston slidable in the container, which piston is provided at its upper side with a displacing member which member in the extreme position of the piston in the container fills up the cavity in the adapter to leave unoccupied only a small space between the inner wall of the adapter and the outer wall of said projection.

Also US—A—3 672 369 describes such a syringe, in which the displacing member closes the passage of the hollow adapter to the hypodermic needle and is made a little tapered just as the cavity in the adapter. Also fig. 13 of Fr—A—242 553 illustrates a syringe of the same type, in which both the cavity in the adapter and the displacing member are made tapered.

The invention provides a hypodermic syringe provided with a container with a hollow adapter connected to the container on which a hypodermic needle can be placed, and with a piston slidable in the container which piston is provided at its upper side with a closed projecting part tapering in the direction of the hypodermic needle to be placed on the adapter, which projecting part in the extreme position of the piston in the container fills up the cavity in the adapter to leave unoccupied only a small space between the inner wall of the adapter and the outer wall of said projection, the top of the projection then closing the passage of the hollow adapter to the hypodermic needle, which is characterized in that the projection on top of the piston is made tapered, its angle of tapering being smaller than the angle of tapering of the inner wall of the adapter.

Such a syringe has the advantage that the remaining space in the adapter when the piston with the displacing member is in the extreme position is very small, so that the loss of injection liquid is minimal, and that a small amount of very small bubbles of air which enter into the container of the hypodermic syringe during sucking in the injection liquid and accumulate on the inner wall of the adapter cannot escape into the needle before the piston reaches it final position.

In a preferred embodiment of a hypodermic syringe according to the invention the piston rod is provided with notches which correspond to the graduation on the cylinder and which enable the measuring of the dosage of an injection liquid to be injected blindly.

Through this provision it is possible that patients with poor eyesight or who are blind can nevertheless inject themselves. Though in general the hypodermic syringe according to the invention will be supplied with a predetermined volume of the container, it is necessary that with this hypodermic syringe variant does can be injected.

The prescribed dose at a time in fact differs from patient to patient. In general the container of the hypodermic syringe is sucked completely, whereupon such an amount of liquid is injected back into the store bottle that the prescribed dose is left in the hypodermic syringe. Even for a blind person it is easy to check in the embodiment just described whether the correct dose is still in the hypodermic syringe before said dose is injected. The invention is further illustrated with the aid of the figures, which relate to an embodiment of a hypodermic syringe according to the invention.

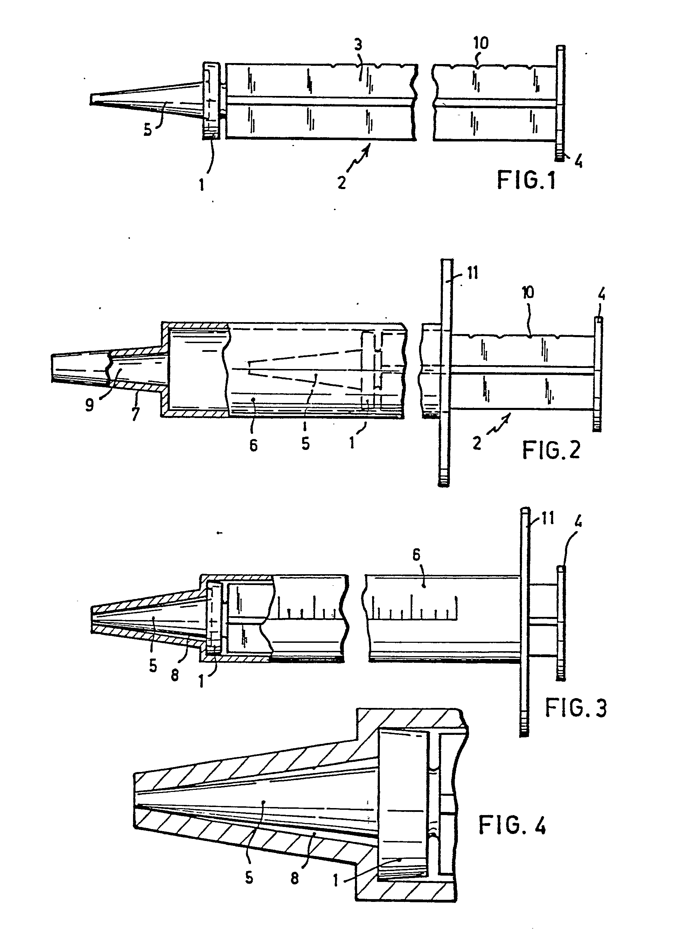

Figure 1 shows a piston of a hypodermic syringe according to the invention in side view.

Figure 2 is a cross-section of a hypodermic syringe according to the invention when the piston is in a position in which injection liquid is present in the container.

Figure 3 shows in cross-section a hypodermic syringe according to the invention with the piston in extreme position.

Figure 4 is enlarged cross-section of the adapter on a hypodermic syringe with in it the projection on the piston in accordance with the invention.

The piston consists of a piston body 1, which can slide fittingly in the cylindrical container 6 with a piston rod 2 which is provided with sides 3 which enable an easy sliding of the piston body through the cylinder. At the end of the piston rod 2 there is an end face 4 to which one pushes if one injects injection liquid. The end face 11 of the cylinder makes it possible that the hypodermic syringe can easily be handled during the injection operation.

The notches 10 make it possible that also a poor-sighted or blind patient can adjust the correct dose and can inject.

In according to the invention on piston body 1 there is a projecting portion 5 which in the extreme position of the piston in the cylinder almost entirely fills the adapter 7 on which the hypodermic needle has to be placed, with the exception of a small space 8.

The cavity 9 in the adapter is tapered at an angle which is a little bit larger than the angle at which the projection on the piston 5 tapers.

## Claim

A hypodermic syringe provided with a container with a hollow adapter connected to the container on which a hypodermic needle can be placed, and with a piston slidable in the container which piston is provided at its upper side with a closed projecting part tapering in the direction of the hypodermic needle to be placed on the adapter, which projecting part in the extreme position of the piston in the container fills up the cavity in the adapter to leave unoccupied only a small space between the inner wall of the adapter and the outer wall of said projection, the top of the projection then closing the passage of the hollow adapter to the hypodermic needle, characterized in that the projection on top of the piston is made tapered, its angle of tapering being smaller than the angle of tapering of the inner wall of the adapter.

## Patentanspruch

Injektionsspritze mit einem Behältnis, an dem ein hohler Adapter zum Aufsetzen einer Kanüle angebracht ist, und mit einem im Behältnis gleitenden Kolben, der an seiner Stirnseite mit einem geschlossenen Vorsprung versehen ist, der in Richtung zu der auf den Adapter aufsetzbaren Kanüle verjüngt ist und bei voll in das Behältnis eingeschobenem Kolben den Hohlraum im Adapter unter Belassung bloss eines geringen Raumes zwischen der Innenwand des Adapters und der Aussenwand des Vorsprunges ausfüllt, wobei die Spitze des Vorsprunges den Durchlass des hohlen Adapters zur Kanüle verschliesst, dadurch gekennzeichnet, dass der Vorsprung an der Stirnseite des Kolbens verjüngt ist, wobei sein Öffnungswinkel kleiner als der Öffnungswinkel der Innenwand des Adapters ist.

## Revendication

Seringue hypodermique équipée d'un conteneur, d'un adaptateur creux connecté au conteneur sur lequel une aiguille hypodermique peut être placée et munie d'un piston coulissant dans le conteneur, lequel piston est équipé sur sa partie supérieure d'une pièce fermée s'éminçant dans la direction de l'aiguille hypodermique devant être placée sur l'adaptateur, laquelle pièce en saillie, lorsque le piston est en position extrême dans le conteneur, comble la cavité dans l'adaptateur, ne laissant vide qu'un tout petit espace entre la paroi extérieure de ladite pièce, le sommet de cette pièce fermant alors le passage de l'adaptateur creux jusqu'à l'aiguille hypodermique, laquelle seringue est caractérisée par le fait que la pièce au sommet du piston est effilée, son angle d'émincement étant plus petit que l'angle d'émincement de la paroi intérieure de l'adaptateur.

FIG.1

FIG.2

FIG.3

FIG. 4